# EUROPEAN PATENT APPLICATION

(11) **EP 1 626 262 A2**
(43) Date of publication of application: **15.02.2006**
(21) Application number: 05253957.4
(22) Date of filing: 24.06.2005
(51) Int. Cl.: G01M 3/32

(54) **A non-destructive leak testing apparatus for ostomy & related drainage pouches**

(30) Priority: 26.06.2004 GB 0414383
(71) Applicant: Air controls and compressors limited, Widnes Cheshire WA8 0SZ (GB)
(72) Inventor: Rose, Mark, Bebington, Wirral CH64 2PT (GB)
(74) Representative: Thomson, Paul Anthony

(57) **Abstract**

Referring to Figure 6, the apparatus of the invention comprises a surface mounted faceplate (12) with a plurality of suction cups (3) embedded in its surface, a clamping device (2) to form an airtight seal between the test article and the faceplate, an airway (9) for the introduction of a positive air pressure into the test article and a means of monitoring airflow to identify leaks. When the aperture of a test article is draped over the clamping device and held on the faceplate by suction a first member of the clamping device (2) moves forward into the test article and is followed by a second member (10) which inserts itself into and expands the circumference of the first member to exceed that of the test article aperture. When the clamping device returns to the faceplate the required airtight seal between the faceplate and the test article is formed.

## Description

### Technical Field

This invention relates to an apparatus for testing pouches intended for the collection of bodily wastes and discharges from colostomy, ileostomy and ureterostomy patients, generally termed ostomy or stoma pouches, for leaks. Ostomy or stoma pouches are attached to the patient by a variety of mechanical and physical (adhesive) means and the present invention concerns an apparatus capable of non-destructively testing for leaks a wide range of pouch types including those that use a pressure sensitive adhesive collar around their aperture for attachment to the patient's stoma region or the stoma pouch coupling collar (wafer).

### Background to the Invention

Ostomy and related drainage pouches are fabricated from any suitable waterproof, flexible but strong sheeting material, which contains an aperture in one side of the pouch to receive the drainage, as described for example in GB785562. These are often constructed from two or more sheets of a flexible, waterproof material, such as Polythene, by a heat sealing process. A drainage aperture which is cut into the pouch which is also fitted with a reinforcing annular collar, of a more rigid material, surrounding the drainage aperture, as for example described in US4388135. Pouch drainage facilities may be incorporated, as described in GB712341, as may venting systems for gas release, as described in EP1101462.

In recent years the preferred pouch has been of the disposable type as, for example, those disclosed in US6652496 or EP1129680. These are often constructed as described above but from bilayer sheets, the outer layer being strong and water soluble and the inner layer being mechanically weak but water insoluble. All such pouches are attached to the stoma area of the patient, either directly or indirectly, often by means of a layer of pressure sensitive adhesive applied to the annular collar surrounding the aperture during manufacture and protected by a strip-away (peel-off) layer prior to use.
The key property of ostomy and related drainage pouches are their ability to collect and retain bodily fluids and discharges of various kinds and any leakage would render the product a liability. Various apparatus and methods have been used in the past to test cavities for leaks by either:
a) injecting air under pressure into the cavity, sealing the cavity and monitoring pressure change with time, by various means; or,
b) evacuating air and monitoring the stability of the ensuing vacuum.
All such methods would require an airtight seal between the ostomy pouch and the test apparatus that is not possible with the modern self-adhesive and disposable pouch, without destroying any self-adhesive property. It is an object of the present invention to provide an apparatus to test ostomy and related drainage pouches for leaks without damaging any self-adhesive property the pouch may possess.

### Summary of Invention

The present invention overcomes the problem of testing ostomy and related drainage pouches for leaks without destroying any self-adhesive properties of the pouch by clamping the test article to the test apparatus mechanically in an air-tight seal, injecting a positive air pressure and subsequently monitoring airflow and/or pressure against time.

### Essential Features of the Invention

According to the present invention there is an apparatus for testing ostomy and related drainage pouches for leaks. The apparatus of the invention consists of a circular profiled aperture cup seal, fabricated from a material possessing resilient elastic properties with an external diameter slightly inferior to the inside diameter of the test article collar, mounted on a planar reinforced surface of the apparatus. The aperture cup seal is able to move forward away from, and subsequently back to the face of the apparatus on the end of a shaft with an airway running through it from the face of the aperture cup seal to a controlled supply of compressed air. Vacuum cups or a vacuum ring are set in the faceplate of the apparatus symmetrically deployed around the aperture cup seal within a distance from its circumference no greater than the distance between the inner and outer circumferences of the test articles reinforced annular collar. When the invention is activated the aperture of a test article is placed over the aperture cup seal, with any adhesive surface of its reinforced collar still protected, and is held in position by the suction exerted on the collar by the surrounding vacuum system. The aperture cup seal is then moved forward, away from the surface of the test apparatus, into the pouch and the diameter of the skirt on the elastic aperture cup seal is increased by insertion of a seal expander mounted co-axially on the aperture seal shaft and capable of independent forward movement. The expanded aperture cup seal is then drawn back to clamp the test article aperture collar to the faceplate of the apparatus of the invention, forming an airtight seal. A positive pressure of air is injected by way of the airway within the aperture cup seal shaft and the air-flow into the pouch is monitored as a function of time. Optionally the pressure within the pouch is monitored and displayed using conventional sensors and an LED display. A satisfactory completion of the test is indicated by an acceptable air-flow at the end of the timed period as shown by the flow meter display. This test procedure and alternative test procedures, some of which are described later, may be initiated manually or automatically and are controlled by electro-pneumatic control circuits.

### Description of Drawings

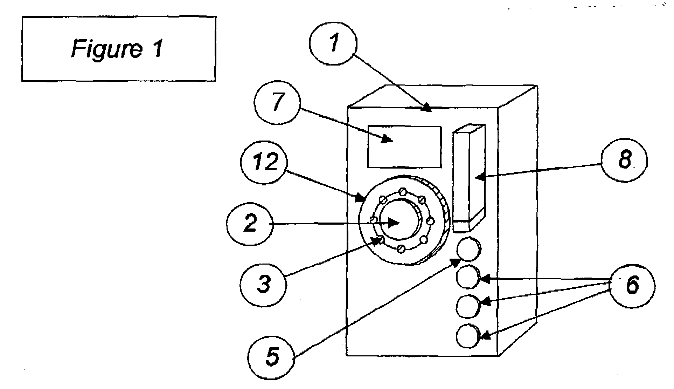

The invention will now be described with the help of figures 1 to 7.
**Figure 1** shows the basic features of the test apparatus in schematic form. The outer casing (1) has a moulded or fabricated aperture cup seal (2) with elastic but resilient walls mounted on its face,and capable of forward motion. The face of the casing in the area underneath and surrounding the aperture cup seal is reinforced with a faceplate (12), which possesses a planar surface containing an array of vacuum cups (3) symmetrically deployed embedded in the surface of the faceplate within an annular area about the aperture cup seal. The on/off switch (5) brings the internal vacuum and air supply systems to readiness or stands them down, the programme switches (6) initiate various testing procedures and the flowmeter (8)and optional LED display (7) provide test information (e.g. flow-rate/pressure/time).

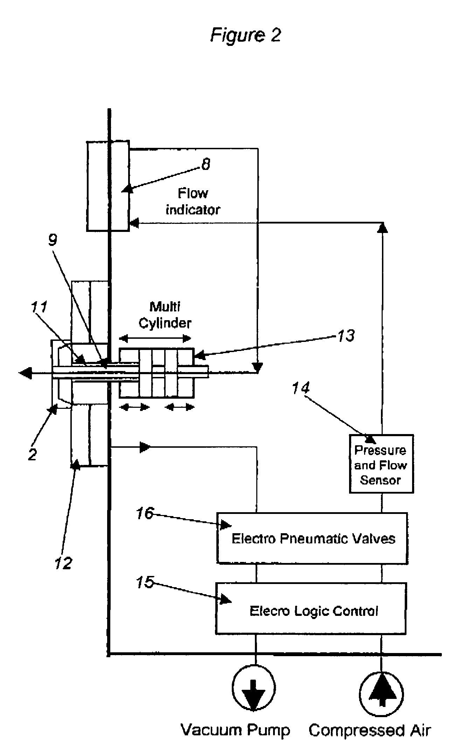

**Figure 2** shows schematically the electro-pneumatic and mechanical components that drive the apparatus. The aperture cup seal(2) is mounted on a shaft (9), incorporating an airway, and rests on a faceplate (12) through which the shaft (9) passes. A shaft (11), which moves coaxially with shaft (9),also passes through the faceplate (12) and both shafts are driven, independently by a multi-cylinder piston (13). The airflow through shaft (9) is caused to pass air pressure sensors (14) and through an airflow meter (8). The apparatus calls upon vacuum and compressed air supplies and is controlled by a process electro-logic controller (15) and electro-pneumatic valves (16)

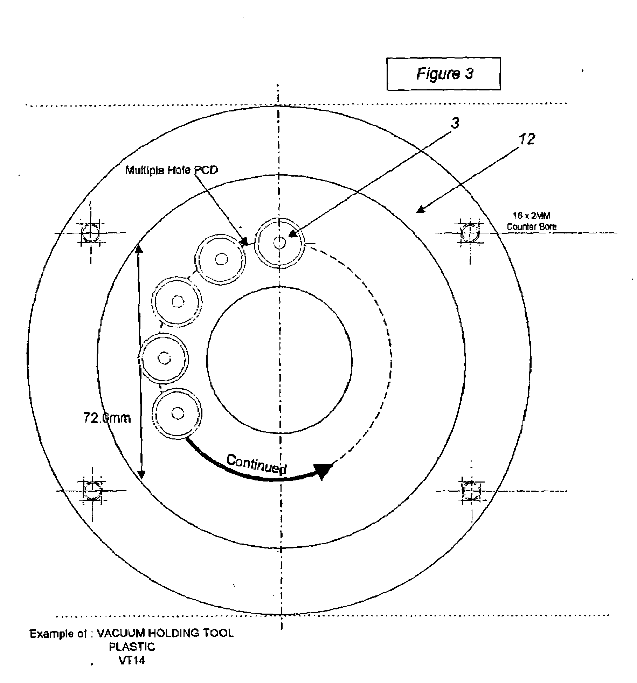

**Figure 3** shows the inter-changeable faceplate (12) which is mounted on the surface of the apparatus in the area around the aperture cup seal in more detail to indicate the deployment of the embedded vacuum cups (3). Only 5 vacuum cups are shown for clarity but any number of rubber vacuum cups between 2 and 25, preferably between 4 and 20 and most preferably between 6 and 16 may be present. The vacuum cups are deployed within an annular area centred on the aperture cup seal (2) with an inside radius of from 6 to 35 mm and an outside radius of from 16 to 55 mm. The size of the vacuum cups may be within the size range 10 to 25 mm diameter, preferably between 15 and 20 mm diameter. The planar faceplate must be thick enough to embed both the vacuum cups and the vacuum supply channels and may be fabricated from any suitable engineering material such as wood, steel, aluminium or engineering plastic. Faceplates with different vacuum cup deployments to suit different aperture cup seals (2) in use are optionally interchangeable.

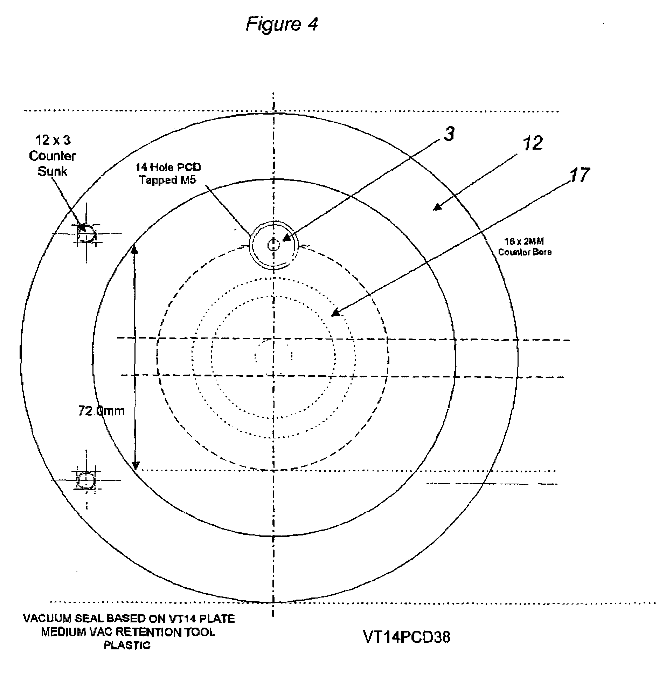

**Figure 4** shows an alternative design of the inter-changeable faceplate (12) which is mounted on the surface of the apparatus in the area around the aperture. Vacuum cups set in the faceplate (3) are augmented with a vacuum ring also set into the surface of the inter-changeable faceplate (12). Vacuum ring and cups are deployed within an annular area (17) centred on the aperture cup seal (2) with an inside radius of from 6 to 35 mm and an outside radius of from 16 to 55 mm.

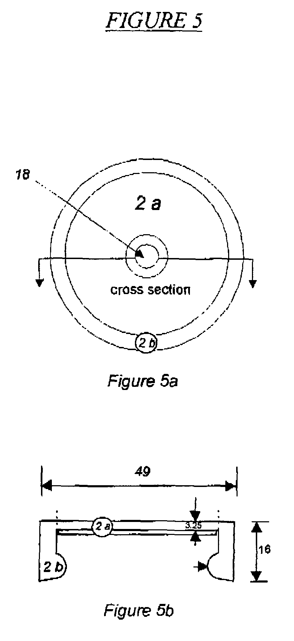

**Figure 5** shows the aperture cup seal (2) in isolation in both frontal (figure 5a) and cross section (figure 5b) views. The circular aperture cup seal may be moulded or fabricated from any resilient material with elastic properties such as RTV silicones, polyurethane or an oil filled nylon (such as Oilon ® available from Bayplastics), or fabricated from a combination of materials. Figure 5b shows one method of fabrication where top section (2a), which incorporates the airway outlet (18) and the attachment to shaft (9), may be constructed of any engineering material such as aluminium, stainless steel or an engineering plastic and the wall or skirt of the cup seal (2b) is constructed from a flexible but resilient material such as RTV silicones, polyurethane resin or oil filled nylon, preferably RTV silicones or oil filled nylon to have an external diameter at rest (i.e. unexpanded) of from 12 to 70 mm. Aperture cup seals of various sizes are optionally interchangeable.

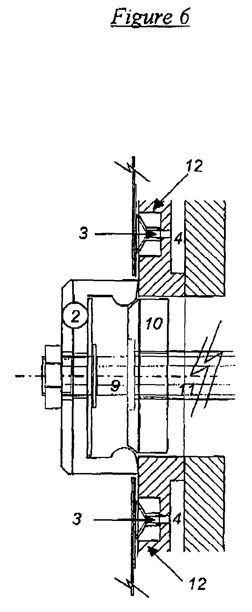

**Figure 6** shows the faceplate (12) of the apparatus in cross-section with the optionally interchangeable aperture cup seal (2) at rest, the vacuum system (3) and its vacuum supply channels (4). Figure 6 also shows the hollow shaft (9) through which a supply of compressed air is brought to the face of the interchangeable aperture cup seal and by means of which the aperture cup seal is moved, by means of a conventional piston system (not shown). Also shown in figure 6 is the interchangeable chamfered edge seal expander disc (10) mounted on shaft (11), which is co-axial with shaft (9) and capable of independent forward movement. The diameter of the seal expander disc (10) should fall within the range 8 to 65 mm, preferably 18 to 60 mm in order to expand the circumference of the aperture cup seal (2) by from 5% to 25% preferably 6% to 22% when inserted into its internal profile. Seal expander discs of different sizes within this range are interchangeable to suit the size of the aperture cup seal in use.

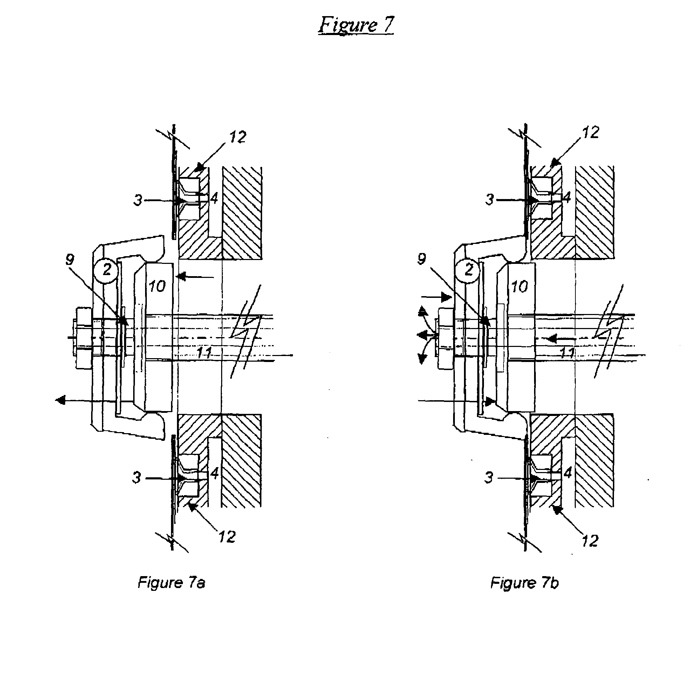

**Figure 7** also shows the faceplate of the apparatus in cross-section at different stages of a test procedure. Figure 7a shows the configuration of the components as the test procedure starts. The aperture cup seal (2) moves forward to clear surface of the apparatus by movement of shaft (9). At the same time or subsequently the chamfered edge expander disc (10) is moved forward on shaft (11), which moves co-axially to shaft (9) and to a greater extent than the movement of shaft (9). The expander disc (10) is forced into the internal profile of the aperture cup seal (2) and increases the effective diameter of the aperture cup seal. The vacuum cups (3) are shown holding the test-piece to be tested in place while the aperture cup seal (2) is raised from the surface of the test apparatus. Figure 7b shows the configuration of the components during the test, with the expanded aperture cup seal (2) pulled back against the faceplate (12) of the apparatus on shaft (9), having trapped the test piece on the apparatus faceplate and formed an airtight seal between the test-piece and the apparatus faceplate. Once an airtight seal is created a positive air pressure is created within the test-piece by injection of compressed air through the airway incorporated into shaft (9). The airflow into the pouch is monitored over a period of time.

### Detailed description of the invention

The apparatus of the present invention may be used to test a wide range of flexible pouches including ostomy drainage pouches for leaks. To improve the readers understanding of how it works several embodiments of the invention will be described.

In a first embodiment of the invention, the apparatus of the invention is used to non-destructively test ostomy and related drainage pouches with self-adhesive collars for leaks without removing the protective film from any pressure sensitive adhesive layer around the aperture collar. Referring to figure 1 the apparatus is switched on (switch (5)) to activate the vacuum holding system (e.g. (3)). Referring now to figures 6 and 7 the reinforced and protected self-adhesive collar around the aperture of the pouch to be tested is placed over the aperture cup seal (2) and retained centrally in position by the vacuum holding system. Any vents or slits intentionally cut into the pouch for functional reasons are reversibly sealed, for the duration of the test, by any known means such as pressure adhesive patches or mechanical clamps (e.g. Brauer Clamp) or clips (e.g. Bulldog Clip). The test routine is initiated by selecting the appropriate switch (6), which activates shaft (9) to move the aperture cup seal (2) forward, away from the faceplate (12) and into the test-piece. Simultaneously or sequentially, shaft (11) moves the seal expander disc (10) forward into the inner profile of the aperture cup seal to expand its diameter. The aperture cup seal with the seal expander inserted is then pulled back onto the surface of the test apparatus to trap the aperture collar of the test-piece and form an airtight seal between it and the test apparatus faceplate (12). Once the aperture cup seal (2) has formed the airtight seal, compressed air is injected into the test-piece through the airway within shaft (9) until a pressure in the range 50 - 185 m bar, preferably 80 - 120 m bar and most preferably 100 - 120 m bar is achieved. The airflow to the pouch, and optionally air pressure in the pouch, is monitored by any of the known means as a function of time in the range 2 - 20 seconds. An unacceptable level of airflow to the pouch after this time period is indicative of a leak in the test-piece. A combination of control valves and a process logic controller, or larger control circuit of the type known in the art controls the entire sequence of events.

In a second embodiment of the invention, which is particularly useful for testing larger aperture pouches, the relative movement and sequence of movements of the aperture cup seal (2) and the seal expander (10) is modified by selecting an appropriate test procedure at the electro-pneumatic control switches (6). The test procedure associated with this second embodiment causes the seal expander disc (10) to be fully inserted into the aperture cup seal (2), beyond the internally encroaching cup seal rim (figure 5) when the apparatus is at rest. Referring now to figures 6 and 7 the reinforced collar around the aperture of the pouch to be tested is placed over the aperture cup seal (2) and retained centrally in position by the vacuum holding system. Any vents or slits intentionally cut into the pouch for functional reasons are reversibly sealed, for the duration of the test, by any known means such as pressure adhesive patches or mechanical clamps (e.g. Brauer Clamp) or clips (e.g. Bulldog Clip). Selection of the appropriate switch (6), activates shaft (9) to move the aperture cup seal (2) forward, away from the faceplate (12) and insert it into the test-piece. Simultaneously or sequentially, shaft (11) pulls the seal expander disc (10) away from the base of the aperture cup seal to expand its diameter by forcing itself between the internally encroaching cup seal rim (figure 5). Subsequently the aperture cup seal, with the seal expander still inserted, is pulled back onto the surface of the test apparatus to trap the aperture collar of the test-piece and form an airtight seal between it and the faceplate (12) of the apparatus of the invention. Once the aperture cup seal (2) has formed the airtight seal, compressed air is injected into the test-piece through the airway within shaft (9) until a pressure in the range 50 - 185 m bar, preferably 80 - 120 m bar and most preferably 100 - 120 m bar is achieved. The airflow to the pouch, and optionally air pressure in the pouch, is monitored by any of the known means as a function of time in the range 2 -20 seconds. An unacceptable level of airflow to the pouch after this time period is indicative of a leak in the test-piece.

In another embodiment, which is particularly useful for leak testing pouches with apertures of less than 30 mm diameter, the seal expander disc (10) is removed from shaft (9) and the procedure relies on compressive forces to buckle the aperture cup seal wall on the faceplate and form the airtight seal. Once the apparatus is switched on and referring now to figures 6 and 7, the reinforced collar around the aperture of the pouch to be tested is placed over the aperture cup seal (2) and retained centrally in position by the vacuum holding system. Any vents or slits cut into the pouch for functional reasons are reversibly sealed, for the duration of the test, by any known means. The test routine is triggered by selecting the appropriate switch (6), which activates shaft (9) to move the aperture cup seal (2) forward, away from the faceplate (12) and inserts it into the test-piece. The aperture cup seal is subsequently pulled back onto the surface of the test apparatus to trap the aperture collar of the test-piece and form an airtight seal between it and the test apparatus faceplate (12). Once the aperture cup seal (2) has formed the airtight seal compressed air is injected into the test-piece through the airway within shaft (9) until a pressure in the range 50 - 165 m bar, preferably 80 - 120 m bar and most preferably 100 - 120 m bar is achieved. The airflow to the pouch, and optionally air pressure in the pouch, is monitored by any of the known means as a function of time in the range 2 - 20 seconds. An unacceptable level of airflow to the pouch after this time period is indicative of a leak in the test-piece.

In yet another embodiment of the invention, which is particularly useful to test non-uniform shaped pouches for leaks, the vacuum holding system (3) is absent or switched off. The test apparatus is switched on (switch (5)) and the pouch aperture is held in position over the aperture cup seal manually or by mechanical means while the appropriate test programme is selected (switch (6)), the aperture cup seal (2) is lifted from the surface to enter the test pouch aperture, expanded by insertion of the seal expander disc (10), and finally pulled back to trap the test pouch collar between itself and the faceplate (12) of the test apparatus, in sequence. The pouch is injected with air by way of the airway within shaft (9) and airflow into the pouch is monitored as a function of time. An unacceptable level of airflow after a time period of 2 - 20 seconds is recorded as a leak.

## Claims

1. An apparatus for testing ostomy and related drainage pouches for leakage comprising: a) a means of holding the aperture collar of the pouch on the surface of the test apparatus; b) a means of clamping the pouch aperture to the surface of the apparatus in an airtight seal; c) a means of injecting a known positive air pressure into the clamped pouch; and, d) a means of monitoring any change in the airflow to the drainage pouch and air pressure within the drainage pouch with time.

2. An apparatus for testing ostomy and related drainage pouches for leaks as claimed in claim 1 wherein the means of holding the pouch aperture collar on the surface of the apparatus is an interchangeable faceplate mounted on the apparatus containing an array of vacuum cups embedded in its outer surface symmetrically deployed about the collar clamping means, which also passes through the faceplate.

3. An apparatus for testing ostomy and related drainage pouches for leaks as claimed in claim 1 or claim 2 wherein a plurality of vacuum cups are embedded symmetrically about the collar clamping device on the otherwise planar surface of an interchangeable faceplate within an annular area centred on the collar clamping device with an inside radius of from 6 to 35 mm and an outside radius of from 16 to 55 mm.

4. An apparatus for testing ostomy and related drainage pouches for leaks as claimed in claim 1 and claim 2 wherein the means of holding the pouch aperture collar on the surface of the apparatus is an interchangeable faceplate mounted on the apparatus containing a circular vacuum slot, in addition to an array of vacuum cups embedded in its outer surface, symmetrically deployed about the collar clamping means, which also passes through the faceplate, such that it falls under the area occupied by the drainage pouch collar.

5. An apparatus for testing ostomy and related drainage pouches for leaks as claimed in claim 1 and claim 4 wherein the means of holding the pouch aperture collar on the surface of the apparatus is an interchangeable faceplate mounted on the apparatus containing a circular vacuum slot, in addition to an array of vacuum cups embedded in its outer surface, symmetrically deployed about the collar clamping means, which also passes through the faceplate and falls within an annular area centred on the collar clamping device with an inside radius of from 6 to 35 mm and an outside radius of from 16 to 55 mm

6. An apparatus for testing ostomy and related drainage pouches for leaks as claimed in claim 1 to claim 5 wherein the means of clamping the pouch to the apparatus surface comprises two members: a) a first circular clamping member fabricated, at least in part, from elastic but resilient material in the profile of a cup attached to the end of a shaft capable of forward and backward movement, emerging through the surface of the apparatus with the wall of the cup resting on the surface of the apparatus; and b) a second clamping member fabricated from a non-elastic material in the form of an oversized internal profile of the first member at the end of a second shaft mounted coaxial on the first member shaft, capable of independent movement and adapted to be forced into the internal profile of the first clamping member to increase the diameter of the first clamping member's wall.

7. An apparatus for testing ostomy and related drainage pouches for leaks as claimed in claim 1 to claim 6 wherein the first clamping member is constructed in the form of a circular shallow cup shape, from an elastic but resilient material or a combination of materials where only the cup wall is formed from an elastic but resilient material, with an outside diameter at rest of between 8 and 65 mm and where the wall of the cup is capable of being reversibly forced out to increase the circumference of the first clamping member wall by from 5% to 25%.

8. An apparatus for testing ostomy and related drainage pouches for leaks as claimed in claim 1 to claim 7 wherein the second clamping member is fabricated from any hard material to form a circular shaped disc the circumference of which is from 5% to 25% greater than the inside circumference of the first clamping member and is chamfered around the edge that first enters the internal profile of the first clamping member.

9. An apparatus for testing ostomy and related drainage pouches for leaks as claimed in claims 1 to claim 8 wherein an electro-pneumatic control circuit causes a sequence of events comprising: a) the first clamping member moves forward; b) the second clamping member moves forward and enters the internal profile of the first member, to expand its diameter, and; c) both clamping members are moved back together to force the expanded wall of the first clamping member to trap anything on the apparatus surface against the apparatus surface, to form an airtight seal.

10. An apparatus for testing ostomy and related drainage pouches for leaks as claimed in claims 1 to claim 8 wherein an electro-pneumatic control circuit causes a sequence of events comprising: a) second clamping member moves forward and enters the internal profile of the first clamping member passing a rim formed on the inside of its wall; b) the first clamping member moves forward; and c) the second clamping member is pulled forward alone to expand the diameter of the first member by lodging between the rim formed on its inside; and c) both clamping members are moved back together to cause the expanded wall of the first clamping member to trap anything on the apparatus surface against the apparatus surface, to form an airtight seal.

11. An apparatus for testing ostomy and related drainage pouches for leaks as claimed in claims 1 to claim 9 wherein an airway runs from the face of the first clamping member through the shaft controlling the movement of said member, air pressure sensing and airflow monitoring system(s), a valving system to a compressed air supply.

12. An apparatus for testing ostomy and related drainage pouches for leaks described herein with reference to Figures 1 - 7.
